# EUROPEAN PATENT APPLICATION

(11) **EP 1 676 537 A1**
(43) Date of publication of application: **05.07.2006**
(21) Application number: 05258078.4
(22) Date of filing: 28.12.2005
(51) Int. Cl.: A61B 17/32, A61B 17/16, A61M 1/00

(54) **Surgical tool with cannulated rotary tip**

(30) Priority: 29.12.2004 US 905350
(71) Applicant: Depuy Mitek, Inc., Norwood, MA 02062 (US)
(72) Inventor: Heneberry, Elizabeth, Westwood Massachusetts 02090 (US); Ranucci, Kevin J., Warwick Rhode Island 02886 (US); Brown, Jeremy, Hope Valley, Derbyshire S32 3YS (GB)
(74) Representative: Mercer, Christopher Paul

(57) **Abstract**

Various devices are provided for cutting and sculpting body tissue, such as cartilage and the like, during a surgical procedure. In an exemplary embodiment, a surgical cutting device is provided which includes an elongate shaft and a rotatable tissue-affecting tip disposed at the distal end thereof. A lumen extends through both the shaft and the tip, and terminates in an opening formed in a distal end of the tissue-affecting tip.

## Description

### FIELD OF THE INVENTION

The present invention relates to surgical instruments, and in particular to powered surgical instruments for cutting and sculpting body tissue.

### BACKGROUND OF THE INVENTION

Powered arthroscopic surgical instruments for cutting body tissue, such as cartilage and the like, typically include a rotating tube that carries a surgical tool on its distal end. The surgical tool cuts the desired body tissue, and the resulting tissue particle debris created by the tool is evacuated from the surgical site.

However, one drawback associated with some surgical cutting and sculpting instruments is that the resulting cut tissue fragments and tissue particle debris can wrap around the shaft of the rotating tool while the tool is in use by the surgeon, or foul the tissue-affecting tip. This not only impairs the surgeon's visability, but it also adds drag to the instrument, and reduces the effectiveness of the tool in cutting tissue.

Accordingly, there remains a need for an improved surgical cutting and sculpting instrument, and in particular a powered surgical instrument to cut and sculpt body tissue.

### SUMMARY OF THE INVENTION

The present invention provides various methods and devices for cutting body tissue, such as cartilage and the like, during a surgical procedure. In one exemplary embodiment, a surgical cutting device is provided which can include an elongate shaft with a rotatable, tissue-affecting tip disposed at its distal end. A lumen extends through the tissue-affecting tip, and terminates at an opening formed on a distal end thereof. Additionally, the surgical tool can have a plurality of cutting flutes formed in a solid, outer surface of the tissue-affecting tip.

While the lumen can have a variety of configurations, in an exemplary embodiment it can be adapted to allow for the evacuation of particles of cut tissue from the surgical environment surrounding the tissue-affecting tip. The lumen can also have a constant diameter throughout the length of the device or, alternatively, the portion of the lumen formed in the shaft and the portion of the lumen formed in the tissue-affecting tip can have different diameters.

In another embodiment of the present invention, a system for cutting body tissue is provided where the shaft and the tissue-affecting tip can rotate as a unit, and the rotation can be powered by a fluid pressure drive system.

### BRIEF DESCRIPTION OF THE DRAWINGS

The invention will be more fully understood from the following detailed description taken in conjunction with the accompanying drawings, in which:
FIG. 1 is a side perspective view of one embodiment of a tissue cutting device of the present invention;
FIG. 2A is a side perspective view of the distal end of the elongate shaft of the tissue cutting device of FIG. 1;
FIG. 2B is a cross-sectional view of the distal end of the elongate shaft of FIG. 2A taken across line B-B;
FIG. 3A is a side perspective view of the unassembled components of a surgical tool which includes the tissue cutting device of FIG. 1; and
FIG. 3B is a side perspective view of the assembled surgical tool of FIG. 3A.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

The present invention provides various methods and devices for cutting and sculpting body tissue, such as cartilage and the like, during a surgical procedure. In an exemplary embodiment, a surgical cutting device is provided which includes an elongate shaft and a rotatable tissue-affecting tip disposed at the distal end thereof. A lumen extends through both the shaft and the tip, and terminates in an opening formed in a distal end of the tissue-affecting tip. One skilled in the art will appreciate that the present invention can be used to cut and sculpt a variety of body tissues, such as cartilage and the like, bone, or any other material used within a human body. One skilled in the art will further appreciate that the surgical cutting device of the present invention can be used in a variety of surgical tools (as will be discussed in more detail below), and therefore can effect a wide range of surgical procedures.

The tissue-affecting tip is useful to treat tissue in a variety of ways. In particular, the tissue-affecting tip can be used to cut, sculpt, and/or shape tissue that it contacts. It is understood that the term "cut" is sometimes used herein to refer to any treatment of tissue by which some quantity of tissue is severed or removed.

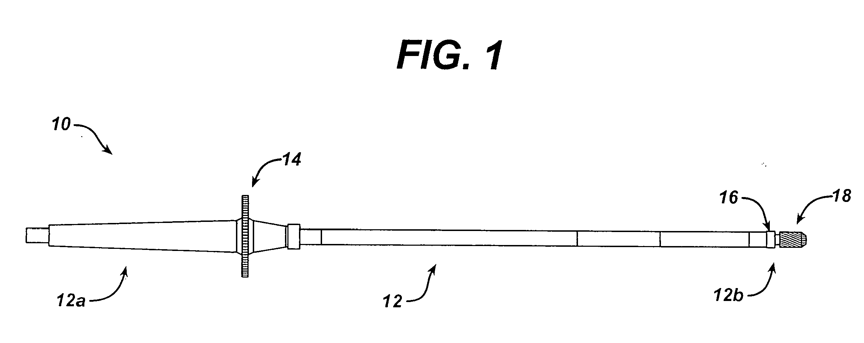

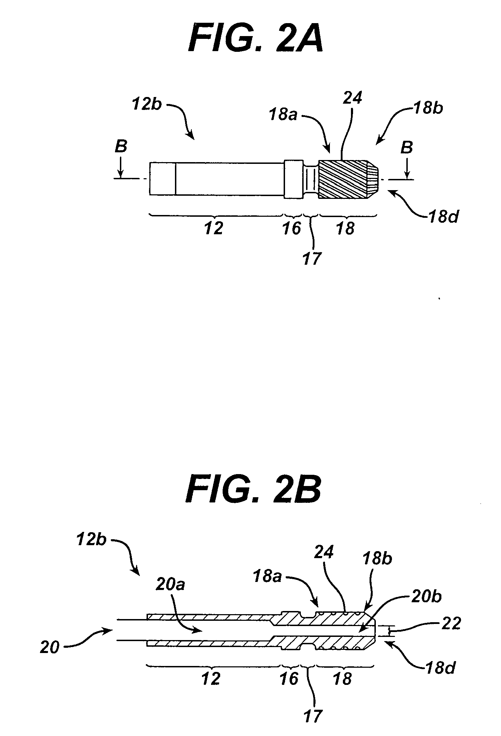

FIGS. 1-2B show an exemplary embodiment of the tissue cutting device 10 of the present invention which has an elongate shaft 12 with proximal and distal ends 12a, 12b, and a tissue-affecting tip 18 disposed on the distal end 12b thereof. A lumen 20 (as shown in FIG. 2B) extends through both the elongate shaft 12 and the tissue-affecting tip 18 and ultimately terminates in an opening 22 in the distal portion 18b of the tip 18.

The shaft 12 can have any configuration which allows a lumen 20 to be formed therein and extend therethrough, such as substantially cylindrical, substantially circular, or substantially ovular. In an exemplary embodiment, the shaft 12 is substantially cylindrical. The shaft 12 can also be configured such that the lumen 20 maintains its integrity during the rotation of the shaft 12, and in an exemplary embodiment, the shaft 12 is elongate and rigidly fixed. Additionally, the shaft 12 can have a variety of features to assist in rotation, such as, for example, one or more bearings 14.

The shaft 12 varies in size depending upon the particular type of surgery with which it is to be used. In one embodiment, the shaft 12 can have a diameter in the range of about 0.02 to 0.20 inch. The shaft 12 can also have a variety of lengths, and in one embodiment, the length of the shaft 12 can be in the range of about 5 to 12 inches.

As noted above, a lumen 20 extends through the shaft 12 and ultimately terminates in an opening 22 in the distal portion 18b of the tissue-affecting tip 18 (as shown in FIG. 2B). The lumen 20 can have a variety of configurations which allow tissue particle debris to be evacuated from the surgical site. In an exemplary embodiment, the lumen 20 is complementary in shape to the shaft 12 (e.g., substantially cylindrical). The lumen 20 can also have a diameter that is less than the diameter of the shaft 12 and the tissue-affecting tip 18, such as, for example, in the range of approximately 0.02 to 0.15 inch. The diameter of the lumen 20 can either be constant throughout the length of the device 10, or, alternatively, a portion of the lumen 20a in the shaft 12 can have a different diameter than a portion of the lumen 20b in the tissue-affecting tip 18. As shown, by way of non-limiting example, the diameter of the portion of the lumen 20a in the shaft 12 has a diameter that is greater than the diameter of the portion of the lumen 20b in the tissue-affecting tip 18, e.g., the diameter of the portion of the lumen 20a within the shaft 12 is in the range of approximately 0.02 to 0.15 inch, and the diameter of the portion of the lumen 20b within the tissue-affecting tip 18 is in the range of approximately 0.02 to 0.08 inch.

Disposed at the distal end 12b of the shaft 12 is a rotatable tissue-affecting tip 18, which can rotate with or independently of the shaft 12. The tip 18 can have any configuration which is suitable to cut, shape, or sculpt tissue, and to permit a cannula to be formed therein. Exemplary shapes of the tissue-affecting tip 18 include, but are not limited to, tapered, conical, spherical, hemispherical, and cylindrical or barrel-shaped. One skilled in the art will understand that the appropriate shape of the tissue-affecting tip 18 will be determined based upon the desired surgical application and the function to be performed by the device. In one embodiment, the tip 18 can be a burr.

In one exemplary embodiment, illustrated in FIGS 1-2B, the tip 18 is substantially cylindrical having a solid outer surface with a plurality of cutting flutes 24 formed thereon. The cutting flutes 24 are substantially helically arranged along the outer surface of the tip 18. One skilled in the art will appreciate that the cutting flutes 24 can have a variety of sizes and geometries depending upon the type of tissue that the tip is to treat and the function that it is to perform with respect to the tissue.

Thus, any number of flutes 24 can be formed on the tissue-affecting tip 18 in a variety of arrangements depending upon the type of cut desired. For example, if precision removal of tissue is desired, there can be anywhere from between about 14 to 18 flutes in a variety of configurations. In one embodiment, the tip has 18 flutes 24 which are equidistant from one another. Further, the flutes 24 have a helix angle of approximately 30° and a rake angle of approximately 3°. While the height of the flutes 24 can vary, the height is typically in the range of about 0.03 to 0.035 inches. The width at the crest of the flutes 24 can also vary, but it is generally in the range of about 0.016 inches. Alternatively, if a more aggressive tissue removal is desired (e.g., cutting of bone), there can be a smaller number of flutes 24 and, as a result, the dimensions of the flutes 24 can increase. One skilled in the art will appreciate that when it is desired to form shallower cutting bites which reduce the likelihood of damage to surrounding tissue and create smaller debris particles, tissue-affecting tip 18 can have a relatively large number of flutes 24.

The distal portion 18b of the tip 18 can have a variety of configurations, including flat, tapered, and radiused. In one embodiment, in which the tip 18 is cylindrical, the distal portion 18b has a full radius.

The tip design, including the flute geometry, and the fact that the tip 18 has a solid outer wall, enhances performance of the tip 18. In use, cut tissue fragments and other debris are directed to the distal most end of tip 18 for evacuation, as described below. The present design tends to resist the accumulation of tissue and other debris between and around the sides of the flutes 24, thus preventing diminution in performance of the device 10 and enhancing visibility of the surgical site for the surgeon.

The tissue-affecting tip 18 can also vary in size, however in an exemplary embodiment, it has a length in the range of approximately 0.2 to about 0.5 inch and a major diameter in the range of about 0.16 to about 0.2 inch.

As noted above, an opening 22 is formed on the distal portion 18b of the tip 18. In an exemplary embodiment, opening 22 is formed on the distal facing surface 18d that is oriented transverse to the longitudinal axis of the tissue-affecting tip 18. In one embodiment, the opening 22 is centrally located on surface 18d. The opening 22 can have a variety of configurations, but it is generally substantially circular and in communication with the lumen 20. In one embodiment, the transition between opening 22 and lumen 20 forms a substantially right angle, as shown in FIG. 2B. It is understood, however, that a taper angle or a reverse angle can be used to form the transition between opening 22 and lumen 20. The opening 22 can also have a variety of sizes depending upon the use of the device 10, however in an exemplary embodiment, the opening 22 has a diameter that is substantially the same as the diameter of lumen portion 20b. Thus, in one exemplary embodiment, the diameter of opening 22 is in the range of approximately 0.02 to 0.08 inch.

The tip 18 can be integral with or removably mated to the shaft 12 in a variety of ways, e.g., press fit, welded, press fit and then welded, or threaded together such that, in an exemplary embodiment, the tip 18 is engaged into the shaft 12 a length which is about the length of one tip 18 diameter. Further, a variety of spacer or washer devices may optionally be disposed between the shaft 12 and tip 18, and in one embodiment, a flange 16 can be used to retain the spacer or washer. In an exemplary embodiment, a transition zone 17 of the tissue-affecting tip 18 that is distal to flange 16 has a reduced diameter. The reduced diameter is useful because it allows a surgeon to access tissue located on the proximal side of the tip 18. The diameter of the transition zone 17 can be in about ¾ the diameter of the tip 18.

One skilled in the art will appreciate that the device of the present invention can have a variety of other features to enhance its tissue cutting and/or sculpting abilities. Such features include, for example, a distal sheath (not shown) which is formed partially around the tissue-affecting tip 18 to partially shield adjacent tissue from the surgical tool.

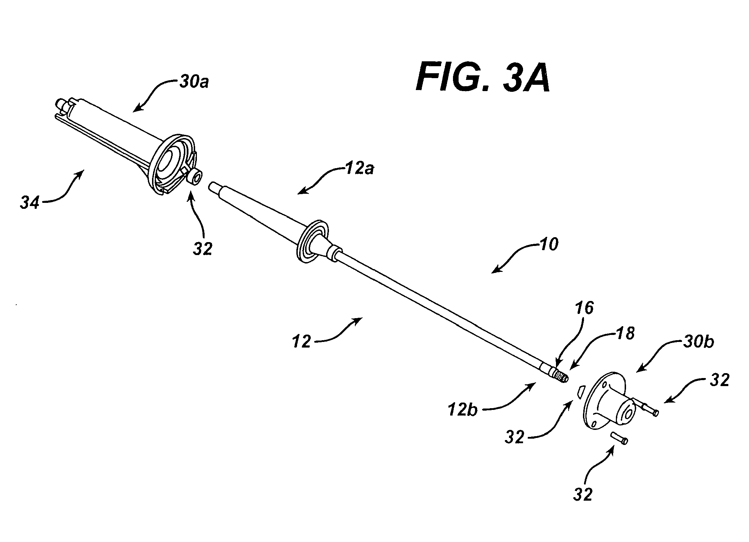

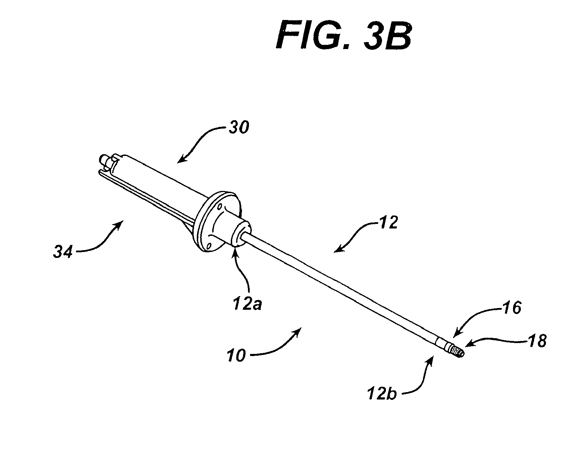

As noted above, the tissue cutting device of FIGS. 1-2B can be used to form a variety of surgical tools for use in many different surgical procedures. By way of non-limiting example, the surgical cutting device 10 is disposed within housing 30 to form surgical tool 34 shown in FIGS. 3A-3B, which can be used, for example, in arthroscopic surgical procedures. While the housing 30 can be virtually any known housing, as shown the housing 30 has front and rear portions 30a, 30b which, when mated together, encase the tissue cutting device 10. The front portion 30a of the housing 30 can have a variety of configurations, however in one embodiment it can include a fluid drive device having a fluid turbojet which delivers a drive fluid to a drive device and causes the tissue cutting device 10 to rotate. The front and rear portions 30a, 30b of the housing 30 can be mated to one another in a variety of ways, so long as they form a casing which surrounds the tissue cutting device 10, and, as shown, the front and rear portions 30a, 30b of the housing 30 are mated to one another by use of a conventional screw and bearing assembly 32. Additionally, the housing 30 can have a variety of components enclosed within or attached thereto, such as, for example, a suction hose (not shown) for evacuation of the tissue particle debris.

In use, the tissue-affecting tip 18 of the surgical tool (such as, for example, surgical tool 34) is introduced through a small incision to the surgical site. An endoscope can also be inserted into the surgical site through a second incision both to provide illumination (from a light source) to the surgical site and to convey an image of the surgical site for viewing by the surgeon. The surgical site can then be inflated with a fluid, which, among other things, irrigates the surgical site and provides a medium by which tissue particles cut by the cutting device 10 are drawn through opening 22, into the lumen 20, and ultimately to a collection tube, container or jar (as will be discussed in more detail below).

Once the surgical site is filled with fluid, the surgeon the maneuvers the distal end 18b of tip 18 to urge the tip 18 against the tissue to be cut and activates the fluid drive device to rotate the tissue cutting device 10 at a desired speed. The surgeon can control the rotational speed and direction (either unidirectional or oscillatory, although an abrader such as surgical tool is typically operated in one direction only) by a variety of known techniques, such as, for example, by foot switches. While the tissue cutting device 10 can rotate over a wide range of speeds, in an exemplary embodiment, the shaft 12 and the tip 18 can rotate at speeds in the range of about 100 rpm to 100,000 rpm. Additionally, the tissue cutting device 10 can deliver a wide range of torques, such as, by way of non-limiting example, a torque of up to 25 oz inches. One skilled in the art will appreciate that a fluid driven device allows a surgeon a better ability to gauge and adjust the torque and as a result only cut the desired tissues. For example, in some applications it may be desirable to configure the device to stop rotation and cutting when it contacts hard tissue, such as bone. In other applications, however, it may be desirable to cut bone.

The surgeon can cut the tissue by moving the surgical tool 34 from side to side and axially, while viewing the surgical site on a monitor. Tissue fragments cut by the tip 18 are broken or chopped up, and are urged into the opening 22 as a result of a positive pressure or suction formed by the rotation of the cutting device 10 and the presence of the irrigation fluid in the surgical site. While this positive pressure is generally suffcient to remove the tissue particles from the surgical site, a vacuum can also optionally be used to enhance suction. Once drawn into the opening 22, the tissue particles and irrigation fluid are transported proximally through the lumen 20 and the surgical tool 34 into a collection container. One skilled in the art will appreciate that the suction formed at the opening 22 of the distal portion 18b of the tissue-affecting tip 18 allows the tool to maintain its integrity and also prevents tissue build-up (and ultimately clogging) of the tip 18.

One skilled in the art will appreciate further features and advantages of the invention based on the above-described embodiments. Accordingly, the invention is not to be limited by what has been particularly shown and described, except as indicated by the appended claims.

## Claims

1. A surgical cutting device, comprising:
an elongate shaft having a proximal end and a lumen extending therethrough along a longitudinal axis thereof;
a rotatable, tissue-affecting tip disposed distally of the elongate shaft, the lumen extending through the tissue-affecting tip, along a longitudinal axis of the tissue-affecting tip, and terminating at an opening formed on a distal end of the tissue-affecting tip; and
a plurality of cutting flutes formed in a solid, outer surface of the tissue-affecting tip,
the lumen being adapted to evacuate particles of cut tissue from within a surgical environment surrounding the tissue-affecting tip.

2. The device of claim 1, wherein the opening is formed on a distal facing surface that is oriented transverse to the longitudinal axis of the tissue-affecting tip.

3. The device of claim 1 or claim 2, wherein the opening has a diameter in the range of 0.02 to 0.08 inch.

4. The device of any one of claims 1 to 3, wherein the lumen has a diameter in the range of 0.02 to 0.08 inch in a portion of the lumen formed within the tissue-affecting tip.

5. The device of claim 4, wherein the lumen has a diameter in the range of 0.02 to 0.15 inch in a portion of the lumen formed within the shaft.

6. The device of any one of claims 1 to 5, wherein the tissue-affecting tip has a radius between a portion of the outer surface of the tissue-affecting tip that extends parallel to the longitudinal axis of the tissue-affecting tip and the distal facing surface of the tissue-affecting tip.

7. The device of claim 6, wherein the tissue-affecting tip is cylindrical and the radius is a full radius.

8. The device of any one of claims 1 to 7, wherein the tissue-affecting tip has approximately 18 flutes, with a rake angle of approximately 3° and a helix angle of approximately 30°.

9. The device of any one of claims 1 to 8, wherein the tissue-affecting tip has a length in the range of 0.2 to 0.5 inch.

10. The device of claim 9, wherein the tissue-affecting tip has a major diameter in the range of 0.16 to 0.2 inch.

11. The device of any one of claims 1 to 10, wherein the shaft and the tissue-affecting tip rotate as a unit.

12. The device of any one of claims 1 to 11, wherein the rotation of the tissue-affecting tip is powered by fluid pressure.

13. The device of any one of claims 1 to 12, further comprising a sheath surrounding at least a portion of the tissue-affecting tip.

14. A system for cutting tissue, comprising:
a fluid-based drive system; and
a surgical cutting device having an elongate shaft with a tissue-affecting tip having a plurality of cutting flutes formed in a solid outer surface thereof and disposed on a distal end of the elongate shaft, and with a lumen extending therethrough along a longitudinal axis and terminating at an opening formed on a distal end of the tissue-affecting tip, the lumen being adapted to evacuate particles of cut tissue from within a surgical environment surrounding the tissue-affecting tip, the tissue-affecting tip being rotatable in response to a force generated by the fluid-based drive system.
